# EUROPÄISCHE PATENTSCHRIFT

(11) **EP 2 048 949 B1**
(45) Veröffentlichungstag und Bekanntmachung des Hinweises auf die Patenterteilung: **27.01.2010**
(21) Anmeldenummer: 07765223.8
(22) Anmeldetag: 18.07.2007
(51) Int. Cl.: A01N 37/14, B27K 3/00, A61K 8/00, A23L 3/00

(54) **KONSERVIERUNGSMITTEL AUF BASIS VON CARBONSÄUREANHYDRIDEN**
PRESERVATIVES BASED ON CARBOXYLIC ANHYDRIDES
CONSERVATEUR À BASE D'ANHYDRIDES CARBOXYLIQUES

(30) Priorität: 29.07.2006 DE 102006035202
(43) Veröffentlichungstag der Anmeldung: 22.04.2009
(73) Patentinhaber: LANXESS Deutschland GmbH, 51369 Leverkusen (DE)
(72) Erfinder: KAULEN, Johannes, 51519 Odenthal (DE); VOGL, Erasmus, 51373 Leverkusen (DE); RITZER, Edwin, 51381 Leverkusen (DE); HOFFMANN, Manfred, 47906 Kempen (DE)
(86) Internationale Anmeldenummer: PCT/EP2007/006361
(87) Internationale Veröffentlichungsnummer: WO 2008/014889

(56) Entgegenhaltungen:
- WO-A-2004/056214
- WO-A-2007/008874
- DE-A1- 1 961 922
- FR-A- 2 877 576
- US-A- 3 097 996
- US-A1- 2002 197 185
- DOBIAS JAROSLAV ET AL: "Properties of polyethylene films with incorporated benzoic anhydride and/or ethyl and propyl esters of 4-hydroxybenzoic acid and their suitability for food packaging" FOOD ADDITIVES AND CONTAMINANTS, Bd. 17, Nr. 12, Dezember 2000 (2000-12), Seiten 1047-1053, XP009100683 ISSN: 0265-203X
- WENG YIH-MING ET AL: "Sorbic anhydride as antimycotic additive in polyethylene food packaging films" LEBENSMITTEL-WISSENSCHAFT AND TECHNOLOGIE, Bd. 30, Nr. 5, 1997, Seiten 485-487, XP002481750 ISSN: 0023-6438 in der Anmeldung erwähnt
- DIVOL B ET AL: "Effectiveness of dimethyldicarbonate to stop alcoholic fermentation in wine" FOOD MICROBIOLOGY, ACADEMIC PRESS LTD, LONDON, GB, Bd. 22, Nr. 2-3, 1. April 2005 (2005-04-01), Seiten 169-178, XP004613725 ISSN: 0740-0020

## Beschreibung

Die Erfindung betrifft die Verwendung von Benzosäureanhydrid und/oder Sorbinsäureanhydrid zur Konservierung von Getränke.

Aus der Literatur sind eine Vielzahl von Carbonsäureanhydriden sowie Methoden zur Herstellung derselben lange bekannt. Eine Anwendung von der o.g. Carbonsäureanhydriden zur direkten Konservierung von Getränke ist bisher nicht beschrieben. US-A-3097996 offenbart die Verwendung von Carbonsäureanhydriden der Formel 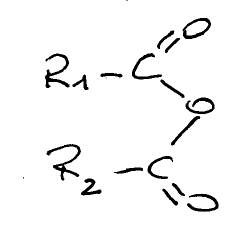 in dem die Reste R1 und R2 unterschiedlich von einander sind, als Zusatz zu Getränken zu deren Schutz vor Befall durch Mikroorganismen. In der WO 2004/056214 ist eine Methode zur Herstellung von Packmaterialien beschrieben, welche immobilisierte antimikrobielle Substanzen, u. a. Säureanhydride, enthalten, die eine biologische Wirkung entfalten sollen indem der Wirkstoff indirekt, nämlich aus dem Packmaterial auf das verpackte Material übergeht und es dadurch konserviert.

Beschrieben sind weiterhin Verpackungsfilme aus Polyethylen, in welche Sorbinsäureanhydrid eingearbeitet wurde. Diese Produkte sollen eine gewisse antifungische Wirkung entfalten. (Weng, Yih-Ming; Chen, Min-Jane; Food Science and Technology, 1997, 30(5), 485-487).

Weiterhin sind aus dem Stand der Technik bereits eine ganze Reihe von Verfahren zur Konservierung von technischen Materialien, Kosmetika oder Lebensmitteln bekannt, wobei der biozide Wirkstoff direkt dem zu konservierenden Produkt zugesetzt wird. Hierbei besteht allerdings weiterhin Verbesserungsbedarf.

Die durch eine Biozidbehandlung in das zu schützende Produkt eingetragenen Biozid-Rückstände werden beispielsweise, besonders in neuerer Zeit, mehr und mehr als problematisch angesehen, und sollten möglichst vermieden werden.

Tatsächlich wurden daher auch schon in der Vergangenheit einige Biozide entwickelt, welche am Wirkort gewissermaßen verschwinden, in dem sie beispielsweise im günstigsten Falle in schon lange bekannte und gut untersuchte und besonders, um ein Vielfaches weniger aktive, Produkte zerfallen. Als Beispiele hierfür seien etwa Stoffe wie Wasserstoffperoxid, Peroxisäuren, Chlor, Diethyldicarbonat oder Dimethyldicarbonat genannt. Es besteht jedoch weiterhin großer Bedarf an neuen mikrobiziden Substanzen welche sich im Medium in relativ unbedenkliche Substanzen zersetzen können und gleichzeitig ein noch besseres Wirkspektrum aufweisen als die bisher verwendeten Substanzen. Insbesondere für den Einsatz in Kosmetika, Pharmaka oder Lebensmitteln kommen nur wenige der nach dem Stand der Technik zur Verfügung stehenden Substanzen in Betracht.

Aufgabe der vorliegenden Erfindung war es demnach, effektive biozide Wirkstoffe bereit zu stellen, die direkt in das zu konservierende Produkt eingearbeitet werden können und dort in weniger aktive Wirkstoffe zerfallen, die beim Einsatz in Getränke als unbedenklich gelten.

Es wurden nun überraschend gefunden, dass Carbonsäureanhydride ausgewählt aus Benzosäureanhydrid und/oder Sorbinsäureanhydrid vorzüglich zur antimikrobiellen Behandlung von Getränken geeignet sind.

Gegenstand der vorliegenden Erfindung ist daher die Verwendung von mindestens einem Carbonsäurcanhydrid ausgewählt aus Benzosäureanhydrid und/oder Sorbinsäureanhydrid als Zusatz zu Getränken zu deren Schutz vor Befall und/oder Zerstörung durch Mikroorganismen.

Vorzugsweise werden die Carbonsäureanhydride direkt in das zu schützende Produkt eingebracht.

Die Carbonsäureanhydride werden in geeigneter Weise direkt in das Getränk eingebracht. Eine gleichmäßige Einbringung ist in der Regel von Vorteil und notwendig. Dies kann beispielsweise durch geeignete Dosierpumpen geschehen. Eine andere Einbringung kann beispielsweise durch Rührwerke oder Mischer erfolgen.

Die erfindungsgemäß einzusetzenden Carbonsäureanhydride können dabei als Reinstoffe oder in Form von Formulierungen dem zu schützenden Produkt zugesetzt werden. Solche Formulierungen enthalten neben den Carbonsäureanhydriden noch ein oder mehrere Lösungsmittel und/oder Formulierhilfmittel. Je nach Löslichkeit der Carbonsäureanhydride kommen als Lösungsmittel Wasser, Ethanol sowie geeignete organische Lösungsmitteln in Frage.

Als Formulierhilfsmittel kommen beispielsweise Tenside, Antischaummittel, Antioxidantien, Stabilisatoren oder inerte organische oder anorganische Hilfsstoffe wie beispielsweise Cellulosefasern in Betracht. Die Carbonsäureanhydride können auch auf adsorptive Medien aufgebracht werden und in dieser Form eingesetzt werden. Beispiele für adsorptive Medien sind Cellulosefasern oder Aktivkohle.

Die Carbonsäureanhydride sind dabei üblicherweise zu 1 bis 90 Gew.-% in diesen Formulierungen enthalten.

Solche Formulierungen können über einen Zeitraum von mehreren Monaten gelagert werden.

Die erfindungsgemäß einzusetzenden Carbonsäureanhydride werden im allgemeinen in einer Menge von 1 bis 100 000 ppm, bevorzugt in einer Menge von 10 bis 10 000 ppm, besonders bevorzugt in einer Menge von 50 bis 5000 ppm, ganz besonders bevorzugt in einer Menge von 100 bis 2000 ppm bezogen auf das zu konservierende Medium eingesetzt.

Durch den erfindungsgemäßen Einsatz der Carbonsäureanhydride gelingt es, Getränke gegen biologische Abbaureaktionen zu stabilisieren. Solche Abbaureaktionen treten z.B. beim Befall mit Mikroorganismen auf.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäß eingesetzten Carbonsäureanhydride gegen Hefen, Bakterien und Pilze.

Es seien beispielsweise Mikroorganismen der folgenden Gattung genannt:
Acetobacter pasteurianus,
Aspergillus, wie Aspergillus niger,
Candida krusei
Chaetomium, wie Chaetomium globosum,
Escherichia, wie Escherichia coli,
Penicillium, wie Penicillium glaucum,
Pseudomonas, wie Pseudomonas aeruginosa,
Rhodotorula wie Rhodotorula rubra
Saccharomyces wie Saccharomyces cervisiae
Staphylococcus, wie Staphylococcus aureus.

Die erfindungsgemäß stabilisierten Getränke zeichnen sich durch eine längere Haltbarkeit aus.

Besonders vorteilhaft ist dabei, dass die erfindungsgemäß eingesetzten Carbonsäureanhydride in die entsprechenden Carbonsäuren hydrolysieren können. Die durch Hydrolyse im Laufe der Zeit durch den Abbau der Carbonsäureanhydride sich bildenden Carbonsäuren sind als Konservierungsstoffe zum Teil lange bekannt, toxikologisch gut untersucht und tragen zusätzlich zu einer Verlängerung der konservierenden Wirkung bei. Zu einer schnellen temporären Abtötung von Keimen kommt damit eine dauerhaft anhaltende Konservierungs-Komponente dazu.

Insbesondere werden Getränke, welche für einen mikrobiologischen Abbau anfällig sind, von den Carbonsäureanhydriden wirkungsvoll konserviert und stabilisiert. So können die so konservierten Getränke nach entsprechender Versiegelung über mehrere Monate bei Raumtemperatur gelagert werden, ohne dass ein mikrobiologischer Befall zu beobachten ist.

Eine solche Methode der Kaltsterilisation hat beispielsweise gegenüber einer Tunnel-Pasteurisation eine Reihe von Vorteilen wie Energieeinsparnis oder apparatetechnische Vorteile. Die Wirksamkeit der erfindungsgemäßen Konservierung ist dabei besser als existierende Verfahren der Kaltsterilisation.

Die erfindungsgemäß einzusetzenden Carbonsäureanhydride eignen sich zum Beispiel in hervorragender Weise als Kaltentkeimungsmittel für stille oder karbonisierte Getränke wie Soft-Drinks, Vitamin-Getränke, Fruchtsaftgetränke, Teegetränke, alkoholische oder entalkoholisierte Wein-Getränke, Fruchtschorlen oder Biere. Bevorzugt werden dazu die Carbonsäureanhydride in Mengen zwischen 10 und 200 ppm zeitnah zur Abfüllung den Getränken zugesetzt. Die Zumischung zu den Getränken erfolgt dabei mit speziellen Dosierpumpen.

Beim Einsatz in Getränken wirken die Carbonsäureanhydride ausgewählt aus Benzosäureanhydrid und/oder Sorbinsäureanhydrid kontrollierend auf eine Reihe von Mikroorganismen wie fermentative Hefen, Schimmel oder fermentative Bakterien. Beispielhaft seien hier etwa genannt Saccharomyces globosum, Saccharomyces diastaticus, Saccharomyces cervisiae, Zygosaccharomyces bailii, Candida crusei, Endomyces lactis, Penicillium glaucum, Acetobacter pasteurianus, Brettanomyces spp, Lactobacillus brevis, Lactobacillus buchneri und viele andere.

Die erfindungsgemäß einzusetzenden Carbonsäureanhydride können in vorteilhafter Weise mit weiteren antimikrobiellen Wirkstoffen vorzugsweise aus der Reihe Dimethyldicarbonat, Diethyldicarbonat, Sorbinsäure und deren Salze, Benzoesäure und deren Salze, Schwefeldioxid und Verbindungen, welche Schwefeldioxid abspalten können, sowie Orthophenylphenol und Propionsäure kombiniert werden.

Besonders bevorzugt sind Mischungen mit Dimethyldicarbonat, Sorbinsäure und deren Salze oder Benzoesäure und deren Salze.

Die erfindungsgemäßen Mischungen enthalten im Allgemeinen mindestens ein Carbonsäureanhydrid der Formel (I) und mindestens einen weiteren antimikrobiellen Wirkstoff im Verhältnis von 1 : 100 bis 100 : 1, bevorzugt von 1 : 10 bis 10 : 1, und besonders bevorzugt von 1 : 1.

Die erfindungsgemäßen Mischungen werden im allgemeinen in einer Menge von 1 bis 100 000 ppm; bevorzugt in einer Menge von 10 bis 10 000 ppm, besonders bevorzugt in einer Menge von 50 bis 5000 ppm, ganz besonders bevorzugt in einer Menge von 100 bis 2000 ppm bezogen auf das zu konservierende Medium eingesetzt.

Die erfindungsgemäßen Mischungen werden beispielsweise direkt in die zu konservierenden Medien eingerührt oder durch Dosierpumpen zugegeben. Die erfindungsgemäßen Mischungen können dabei direkt oder in Form von Formulierungen dem zu schützenden Produkt zugesetzt werden. Solche Formulierungen enthalten neben den Bestandteilen der Mischung noch ein oder mehrere Lösungsmittel und/oder Formulierhilfmittel. Als Lösungs- und Formulierhilfsmittel kommen die oben genannten Verbindungen in Frage. Die Lösungs- und Formulierhilfsmittel werden in den für diese Stoffe üblichen Mengen eingesetzt.

Die folgenden Beispiele sollen den Gegenstand der Erfindung verdeutlichen, ohne diesen jedoch darauf zu beschränken.

### Beispiel 1

Erfrischungsgetränke wurden mit den jeweils angegebenen Mikroorganismen kontaminiert. Die zur Entkeimung der Getränke notwendige Wirkstoff-Konzentration wurde durch Abtöteversuche ermittelt.

### Nährmedien: Hefen Orangenserum-Agar (Fa. Oxoid CM 657)

**Substanzen:** Die eingesetzten Substanzen wurden unverdünnt oder als ethanolische oder butanolische Lösungen (z.B. 2,0 V/V %) oder Suspensionen eingesetzt. Dimethyldicarbonat, Na-Benzoat, K-Sorbat, Sorbinsäureanhydrid und Benzoesäureanhydrid wurden nach bekannten Methoden synthetisiert oder waren käuflich verfügbar. Die jeweiligen Lösungen wurden jeweils frisch angesetzt (Alkoholyse).

### Geräte: 50 ml Gewebekulturflaschen der Fa. Greiner, Mullkompressen der Fa. Holthans, Zählkammer nach Thoma der Fa. Brand.

Verwendet wurden grundsätzlich sterile Reagenzien und Geräte. Neben sterilen Einmalartikeln wurden die nötigen Geräte und Reagenzien vor Verwendung durch geeignete Maßnahmen sterilisiert. Maßnahmen der Sterilisation waren Hitzebehandlung im Trockenschrank (min. 8 Stunden ca. 120°C) oder im Autoklav (min. 15 Minuten ca. 120°C).

### Durchführung:

### Abfüllen der Getränke:

Die Getränkesubstrate wurden vor der Durchführung des Versuches in Gewebekulturflaschen abgefüllt. Die Getränkemenge betrug in der Regel 40 mL. Das Abfüllen wurde unter sterilen Bedingungen durchgeführt. Zu diesem Zeitpunkt konnten auch bereits die persistenten Konservierungsmittel eingebracht werden.

### Herstellung der Keimsuspension:

Die optimal gewachsenen Kulturen (in Gewebekulturflaschen Greiner; 50 mL, 25 cm² oder in Schrägagarröhrchen) wurden mit bis zu 30 mL (je nach Prüfkeim) NaCl-Lösung benetzt (evtl unter Zugabe von ca. 3 Tropfen Tween 80), abgeschwemmt oder mittels Glasperlen (je nach Keim) intensiv abgerollt und bei Bedarf filtriert. Alternativ konnten auch Flüssigkulturen verwendet werden. Eine orientierende Keimzahlbestimmung war unter Zuhilfenahme eines Mikroskopes und einer Thoma-Kammer möglich. Eine genaue Keimzahlbestimmung der eingesetzten Keim-Lösung wurde nach dem Koch'schen Plattengußverfahren durchgeführt.

### Zugabe der Keimsuspension:

Je nach vorgegebener Keimeinsaat (z.B. 50 - 500 Keime / mL Substrat) wurden die entsprechenden Volumina an Keimlösung (Original oder Verdünnung) zugesetzt und die Probe intensiv geschüttelt.

### Zugabe der Wirkstoffe:

Anschließend erfolgte die Zugabe der Wirkstoff Dosagen, zugegeben als reine Substanz oder als entsprechende alkoholische Lösung oder Suspension im Fall von Dimethyldicarbonat und der Carbonsäureanhydride. Über einen Zeitraum von ca. 20 Sekunden wurden die Proben sofort intensiv geschüttelt.

### Lagerung

Die Getränkeproben wurden bei 26 °C ± 2 °C Wachstumstemperatur gelagert.

### Auswertung

Eine Trübung bei ehemals klaren Getränken weist auf eine Kontamination hin. Bei trüben, fruchtfleischhaltigen Getränken war eine visuelle Auswertung nur bedingt möglich. Daher wurde hier zur Auswertung eine Keimzahlbestimmung in Agar durchgeführt.

**Tabelle 1**

| **Wirksamkeit von Benzoesäureanhydrid in Apfelsaft gegen *Saccharomyces cerevisiae*** | | | | | |
|---|---|---|---|---|---|
| Substanzen: | | | Benzoesäureanhydrid 100, 150, 250 mg/L | | |
| | | | Dimethyldicarbonat 250 mg/L | | |
| | | | Natriumbenzoat 177 mg/L | | |
| | | | | | |
| Testkeim: Sacharomyces cerevisiae | | | | | |
| | | | | | |
| Substrat: | Apfelsaft | | | | |
| pH-Wert: | 3,5 und 6,0 | | | | |
| | | | | | |
| Einfluss der Testsubstanzen auf die Wirksamkeit nach 24 Stunden Kontaktzeit bei 20°C | | | | | |
| | | | | | |
| Keimeinsaat pro mL Substrat: | | | 56.000 cfu/mL | | |

| | | | Keimzahl nach 24 h (doppelt durchgeführt) | | Visuelle Bewertung nach 4 Wochen |
|---|---|---|---|---|---|
| Kontrolle (kein Wirkstoff) | | pH 3.5 | >3x10⁶ | 2.7x10⁶ | + |
| | | pH 6.0 | 2.1x10⁶ | 2.5x10⁶ | + |
| Dimethyldicarbonat | 250 mg/L | pH 3.5 | <10 | 160 | + |
| Nicht erfindungsgemäß | | pH 6.0 | 2060 | 830 | - |
| Benzoesäureanhydrid | 100 mg/L | pH 3.5 | <10 | <10 | - |
| | | pH 6.0 | 100 | 45 | + |
| Benzoesäureanhydrid | 150 mg/L | pH 3.5 | <10 | <10 | - |
| | | pH 6.0 | ca. 10 | ca. 10 | + |
| Benzoesäureanhydrid | 250 mg/L | pH 3.5 | <10 | <10 | - |
| | | pH 6.0 | <10 | <10 | - |
| Na-benzoat | 177 mg/L | pH 3.5 | 6.8x10⁵ | 5.3x10⁵ | + |
| Nicht erfindungsgemäß | | pH 6.0 | 2.6x10⁶ | 2.2x10⁶ | + |

| | | | | | |
|---|---|---|---|---|---|
| - = unvergoren + = vergoren | | | | | |

### Beispiel 2

Zur Durchführung der Versuche wurde wie in Beispiel 1 angegeben verfahren.

**Tabelle 2**

| **Wirksamkeit von Sorbinsäureanhydrid in Apfelsaft gegen *Saccharomyces cerevisiae*** | | | | |
|---|---|---|---|---|
| Substanzen: | | Sorbinsäureanhydrid 50, 100, 250 mg/L | | |
| | | Dimethyldicarbonat 250 mg/L | | |
| | | Natriumbenzoat 177 mg/L | | |
| | | | | |
| Testkeim: Sacharomyces cerevisiae | | | | |
| | | | | |
| Substrat: | Apfelsaft | | | |
| | | | | |
| Einfluss der Testsubstanzen auf die Wirksamkeit nach 24 Stunden Kontaktzeit bei 20°C | | | | |
| | | | | |
| Keimeinsaat pro mL Substrat: | | | 56.000 cfu/mL | |

| | | Keimzahl nach 24 h (doppelt durchgeführt) | | Visuelle Bewertung nach 4 Wochen |
|---|---|---|---|---|
| Kontrolle (kein Wirkstoff) | | 1.8x10⁶ | 1.1x10⁶ | + |
| Dimethyldicarbonat | 250 mg/L | <10 | <10 | + |
| Nicht erfindungsgemäß | | | | |
| Sorbinsäureanhydrid | 50 mg/L | <10 | <10 | - |
| Sorbinsäureanhydrid | 100 mg/L | <10 | <10 | - |
| Sorbinsäureanhydrid | 250 mg/L | <10 | <10 | - |
| Na-benzoat | 177 mg/L | 1.3x10⁵ | 1.1x10⁵ | + |
| Nicht erfindungsgemäß | | | | |

### Beispiel 3

Zur Durchführung der Versuche wurde wie in Beispiel 1 angegeben verfahren.

**Tabelle 3**

| **Wirksamkeit von Sorbinsäureanhydrid in Apfelsaft gegen *Hefe-Mix*** | | | | |
|---|---|---|---|---|
| Substanzen: | | Sorbinsäureanhydrid 50, 100, 250 mg/L | | |
| | | Dimethyldicarbonat 250 mg/L | | |
| | | Natriumbenzoat 177 mg/L | | |
| | | | | |
| Testkeim: Mischung unterschiedlicher problematischer Hefen aus Getränkeabfüllstraße | | | | |
| | | | | |
| Substrat: | Apfelsaft | | | |
| | | | | |
| Einfluss der Testsubstanzen auf die Wirksamkeit nach 24 Stunden Kontaktzeit bei 20°C | | | | |
| | | | | |
| Keimeinsaat pro mL Substrat: | | 56.000 cfu/mL | | |

| | | Keimzahl nach 24 h (doppelt durchgeführt) | | Visuelle Bewertung nach 4 Wochen |
|---|---|---|---|---|
| Kontrolle (kein Wirkstoff) | | 6.6x10⁶ | 8.1x10⁶ | + |
| Dimethyldicarbonat | 250 mg/L | 6.8x10⁶ | 5.7x10⁶ | + |
| Nicht erfindungsgemäß | | | | |
| Sorbinsäureanhydrid | 50 mg/L | 2.6x10⁵ | 1.8x10⁵ | + |
| Sorbinsäureanhydrid | 100 mg/L | 9.2x10⁴ | 9.4x10⁴ | + |
| Sorbinsäureanhydrid | 250 mg/L | 985 | 1080 | - |
| Na-benzoat | 177 mg/L | 1.3x10⁵ | 1.1x10⁵ | + |
| Nicht erfindungsgemäß | | | | |

## Patentansprüche

1. Verwendung von mindestens einem Carbonsäureanhydrid ausgewählt aus der Gruppe bestehend aus Benzoesäureanhydrid und Sorbindsäureanhydrid als Zusatz zu Getränken zu deren Schutz vor Befall und/oder Zerstörung durch Mikroorganismen.

2. Verwendung gemäß Anspruch 1, **dadurch gekennzeichnet, dass** die Carbonsäureanhydride in einer Menge von 1 bis 100 000 ppm bezogen auf das zu schützende Getränk eingesetzt werden.

## Claims

1. Use of at least one carboxylic anhydride selected from the group consisting of benzoic anhydride and sorbic anhydride as additive for beverages for protecting them against attack and/or destruction by microorganisms.

2. Use according to Claim 1, **characterized in that** the carboxylic anhydrides are employed in an amount of from 1 to 100 000 ppm, based on the beverage to be protected.

## Revendications

1. Utilisation d'au moins un anhydride d'acide carboxylique choisi dans le groupe constitué par l'anhydride de l'acide benzoïque et l'anhydride de l'acide sorbique comme additif à des boissons en vue de leur protection contre une attaque et/ou une dégradation par des microorganismes.

2. Utilisation selon la revendication 1, **caractérisée en ce que** les anhydrides d'acide carboxylique sont utilisés en une quantité de 1 à 100 000 ppm par rapport à la boisson à protéger.
